# EUROPÄISCHE PATENTSCHRIFT

(11) **EP 3 678 977 B1**
(45) Veröffentlichungstag und Bekanntmachung des Hinweises auf die Patenterteilung: **19.10.2022**
(21) Anmeldenummer: 18766210.1
(22) Anmeldetag: 06.09.2018
(51) Int. Cl.: B67B 3/00, A61L 2/20

(54) **VORRICHTUNG ZUM BEHANDELN VON BEHÄLTERVERSCHLÜSSEN**
DEVICE FOR THE TREATMENT OF CONTAINER CLOSURES
DISPOSITIF POUR LE TRAITEMENT DES BOUCHONS POUR DES RÉCIPIENTS

(30) Priorität: 06.09.2017 DE 102017120557
(43) Veröffentlichungstag der Anmeldung: 15.07.2020
(73) Patentinhaber: KRONES AG, 93073 Neutraubling (DE)
(72) Erfinder: SCHOENFELDER, Markus, 93073 Neutraubling (DE); BUCHHAUSER, Klaus, 93073 Neutraubling (DE); GRÜNWALD, Angela, 93073 Neutraubling (DE); KLEPATZ, Sebastian, 93073 Neutraubling (DE)
(74) Vertreter: Nordmeyer, Philipp Werner
(86) Internationale Anmeldenummer: PCT/EP2018/073936
(87) Internationale Veröffentlichungsnummer: WO 2019/048516

(56) Entgegenhaltungen:
- EP-A1- 0 329 632
- WO-A1-2013/068379
- WO-A1-2017/064186

## Beschreibung

### Technisches Gebiet

Die vorliegende Erfindung betrifft eine Vorrichtung zum Behandeln von Behälterverschlüssen, bevorzugt zum Desinfizieren von Behälterverschlüssen in einer Getränkeabfüllanlage.

### Stand der Technik

Vorrichtungen zum Behandeln von Behälterverschlüssen, bevorzugt zum Desinfizieren von Behälterverschlüssen in einer Getränkeabfüllanlage, sind bekannt.

Hierbei ist es bekannt, in aseptischen Getränkeabfüllanlagen trockene

Behälterverschlussdesinfektionssysteme einzusetzen, mittels derer die auf die dann befüllten

Behälter aufzusetzenden Behälterverschlüsse desinfiziert werden. Die

Behälterverschlussdesinfektionssysteme sind beispielsweise mit einem geneigten Kanal versehen, in welchem die zu desinfizierenden Behälterverschlüsse aufgrund der Erdbeschleunigung entlang gleiten und dabei mit einem Desinfektionsmittel beaufschlagt werden. Diese Art von Vorrichtungen zum Desinfizieren von Behälterverschlüssen baut hoch auf und erfordert daher große Hallenhöhen von bis zu 10 m Deckenhöhe. Um einen erforderlichen Durchsatz an Behälterverschlüssen zu erzielen, muss stets eine Vielzahl von Behälterverschlüssen zeitgleich durch den Kanal geführt werden. Dadurch kann es durch den Staudruck der durch den Kanal geführten Behälterverschlüsse zu einem Verkeilen oder Verklemmen kommen.

Entsprechend sind Vorrichtungen bekannt, in welchen statt der Behälterverschlussförderung via Gravitation im Wesentlichen horizontale Verschlussführungen vorgesehen sind, entlang welchen die Behälterverschlüsse mittels einer Transportvorrichtung durch ein Desinfektionsmedium bewegt werden.

Aus der EP 2 039 439 A1 ist eine Vorrichtung zum Desinfizieren von Behälterverschlüssen bekannt, welche einen horizontal angeordneten, spiralförmig ausgebildeten Transportpfad aufweist. Die Behälterverschlüsse werden entlang des Transportpfads mittels einer Vielzahl von

Verschiebekörpern von außen nach innen bewegt, wobei jeder Behälterverschluss jeweils zwischen zwei Verschiebekörpern geführt wird. Um verschiedene Arten oder Typen von Behälterverschlüssen in dieser Vorrichtung behandeln zu können, ist entweder der Abstand zwischen zwei benachbarten Verschiebekörpern jeweils auf den neu zu behandelnden Behälterverschluss anzupassen, oder aber, wenn ein Umbau vermieden werden soll, auf den größten Behälterverschlusstyp einzustellen. Dadurch kann es jedoch insbesondere beim Fördern kleinerer Behälterverschlüsse zu einem Kippen oder Verkeilen von Behälterverschlüssen kommen.

Die JPH04-106017 A zeigt eine Vorrichtung zum Behandeln von Behälterverschlüssen, wobei die Behälterverschlüsse entlang einer horizontal angeordneten, spiralförmig ausgebildeten Transportbahn mittels einer darunter angeordneten drehenden Transportscheibe von innen nach außen transportiert werden. Durch das Abrollen der Behälterverschlüsse entlang der Transportbahn kann es zu einem Kippen und Verkeilen von Behälterverschlüssen kommen, wodurch die Behandlung der Behälterverschlüsse unterbrochen und die Verkeilung behoben werden muss.

Die WO 2017/064186 A1 beschreibt eine Vorrichtung gemäß dem Oberbegriff des Anspruchs 1 und zwar eine Zwischenspeichereinrichtung für vereinzelte Gegenstände wie Verschlüsse mit einem spiralförmigen Speicherweg.

### Darstellung der Erfindung

Ausgehend von dem bekannten Stand der Technik ist es eine Aufgabe der vorliegenden Erfindung, eine verbesserte Vorrichtung zum Behandeln von Behälterverschlüssen, bevorzugt zum Desinfizieren von Behälterverschlüssen in einer Getränkeabfüllanlage, bereitzustellen.

Die Aufgabe wird durch eine Vorrichtung zum Behandeln von Behälterverschlüssen, bevorzugt zum Desinfizieren von Behälterverschlüssen in einer Getränkeabfüllanlage, mit den Merkmalen des Anspruchs 1 gelöst. Vorteilhafte Weiterbildungen ergeben sich aus den Unteransprüchen, der Beschreibung und den Figuren.

Entsprechend wird eine Vorrichtung zum Behandeln von Behälterverschlüssen, bevorzugt zum Desinfizieren von Behälterverschlüssen in einer Getränkeabfüllanlage, vorgeschlagen, umfassend eine um eine vertikale Drehachse drehbare Transportscheibe zum Transportieren der Behälterverschlüsse und eine oberhalb der Transportscheibe angeordnete, spiralförmige Verschlussführung zum seitlichen Führen der Behälterverschlüsse. Erfindungsgemäß weist die Verschlussführung eine Deckenführung zum Führen der Behälterverschlüsse von oben auf.

Dadurch, dass die Verschlussführung eine Deckenführung zum Führen der Behälterverschlüsse von oben aufweist, ist ermöglicht, die Behälterverschlüsse stets in einer Position mit korrekter Orientierung, in welcher ein optimales Behandlungsergebnis erzielt werden kann, zu halten. Insbesondere kann ein Kippen und/oder Verkeilen von Behälterverschlüssen während des Transports mittels der Transportscheibe vermieden werden. Die Deckenführung führt die Behälterverschlüsse auf der der Transportscheibe gegenübergelegenen Seite der Behälterverschlüsse. Erfährt ein entlang der Verschlussführung transportierter Behälterverschluss ein Kippmoment, welches ihn aus seiner korrekt orientierten Position heraus kippen würde, so ist durch die Deckenführung eine aus dem Kippmoment resultierende Kippbewegung des Behälterverschlusses begrenzt oder gar gänzlich vermieden. Mit anderen Worten ist dadurch ermöglicht, die Behälterverschlüsse sowohl seitlich durch die Verschlussführung als auch von oben mittels der Deckenführung zu führen. Folglich ist ein Eingrenzen der Behälterverschlüsse von vier Seiten ermöglicht. Damit verbleibt lediglich die Bewegungsrichtung entlang der Transportbahn als Bewegungsmöglichkeit für die Behälterverschlüsse.

Bevorzugt sind die Verschlussführung und/oder die Transportscheibe und/oder die Deckenführung im Wesentlichen derart angeordnet, so dass eine Führung der Behälterverschlüsse entlang der vorgegebenen Führungsbahn erfolgt.

Bevorzugt weist eine Ebene, welche durch die so gebildete Führungsbahn ausgebildet wird beziehungsweise auf der die Führungsbahn liegt, gegenüber der Horizontalen eine Neigung auf, bei welcher eine entsprechend des Neigungswinkels auf die Behälterverschlüsse wirkende Hangabtriebskraft derart gering ist, dass ein Transport der Behälterverschlüsse mittels der Transportscheibe nicht oder nur unwesentlich beeinträchtig wird. Bevorzugt beträgt der Neigungswinkel zur Horizontalen 0° bis 5°, besonders bevorzugt 0° bis 3°. Ganz besonders bevorzugt sind die Verschlussführung, die Transportscheibe und/oder die Deckenführung im Wesentlichen im Wesentlichen horizontal ausgerichtet, so dass eine Führung der Behälterverschlüsse entlang einer im Wesentlichen horizontalen Führungsbahn erfolgt.

Unter "vertikal" wird entsprechend in Richtung der Erdbeschleunigungsrichtung sowie eine Abweichung von der Erdbeschleunigungsrichtung mit ± 5°, bevorzugt ± 3°, besonders bevorzugt ohne Abweichung, verstanden.

In einer weiteren bevorzugten Ausgestaltung ist die durch die Transportscheibe ausgebildete Ebene horizontal angeordnet und es besteht keinerlei Neigung gegenüber der Horizontalen. Mit anderen Worten liegt die Transportscheibe "im Wasser" und ist genau in der Horizontalen ausgerichtet. Mit der Transportscheibe ist dann bevorzugt auch die Verschlussführung und die Deckenführung horizontal ausgeführt, so dass diese ebenfalls "im Wasser" ausgerichtet sind.

Gemäß der Erfindung ist die Deckenführung höhenverstellbar, bevorzugt stufenlos höhenverstellbar. Die Deckenführung weist ein spiralförmig ausgebildetes, flaches Führungsblech auf, welches an einer in Richtung der Drehachse höhenverstellbaren Halterung befestigt ist. Dadurch kann ein Abstand zwischen der Transportscheibe
und der Deckenführung auf die Höhe des zu behandelnden Behälterverschluss angepasst werden, so dass die Vorrichtung zur Behandlung unterschiedlicher Behälterverschlussarten verwendet werden kann, ohne dass es aufgrund eines zu hohen Abstands zwischen Deckenführung und Behälterverschluss zu einem Kippen oder Verkeilen kommen kann. Bevorzugt wird die Höhe der Deckenführung derart eingestellt, dass zwischen den zu befördernden Behälterverschlüssen und der Deckenführung ein vorgegebener Abstand, bevorzugt ein Abstand von 1 mm bis 10 mm, besonders bevorzugt von 2 mm bis 8 mm, ganz besonders bevorzugt von 4 mm bis 6 mm, vorliegt. Mit anderen Worten wird die Höhe der Deckenführung derart eingestellt, dass ein zwischen der Transportscheibe und der Deckenführung vorliegender Höhenabstand der Höhe des jeweiligen zu behandelnden Behälterverschlusstyps plus den vorgenannten Abstand entspricht.

Unter "höhenverstellbar" wird in diesem Sinne ein Verstellen in Richtung der vertikalen Drehachse verstanden.

Ein besonders einfacher, robuster und kostengünstiger Aufbau kann erzielt werden, wenn die Verschlussführung gemäß einer weiteren bevorzugten Ausführungsform feststehend ist.

Um zu ermöglichen, dass ein Behandlungsmedium, bevorzugt ein Desinfektionsmedium, besonders bevorzugt H2O2, während des Transports der Behälterverschlüsse an die Behälterverschlüsse gelangen kann, ist die Transportscheibe gemäß einer weiteren bevorzugten Ausführungsform perforiert, wobei die Transportscheibe bevorzugt eine Vielzahl von bevorzugt gleichmäßig verteilten Perforationen aufweist.

Wenn die Verschlussführung einer weiteren bevorzugten Ausführungsform entsprechend derart ausgebildet ist, dass in Bezug auf die Drehachse die Behälterverschlüsse von radial innen nach radial außen transportierbar sind, kann ein besonders gutes Behandlungsergebnis erzielt werden. Durch den Transport der Behälterverschlüsse von radial innen nach außen wird der Abstand von Behälterverschluss zu Behälterverschluss mit der Verlagerung der Bahn nach außen immer größer, da mit steigenden Durchmesser auch der Umfang steigt. Mithin wird der Abstand zwischen den einzelnen Behälterverschlüssen immer größer, was einem Aufstauen von Behälterverschlüssen entgegenwirkt.

Gemäß einer weiteren bevorzugten Ausführungsform ist eine Drehgeschwindigkeit der Transportscheibe einstellbar, wobei bevorzugt durch ein Einstellen der Drehgeschwindigkeit der Transportscheibe eine Verweilzeit eines zu behandelnden Behälterverschlusses einstellbar ist. Dadurch ist ermöglicht, dass die zu behandelnden Behälterverschlüsse stets eine erforderliche Dauer über in der Vorrichtung verweilen und der Behandlung, bevorzugt der Desinfektion, unterzogen werden können. Zudem kann dadurch eine Taktung, mit welcher die Behälterverschlüsse an das Ende der Verschlussführung gelangen, eingestellt werden.

Gemäß einer weiteren bevorzugten Ausführungsform haust ein Gehäuse, bevorzugt ein abgedichtetes Gehäuse, die Transportscheibe, die Verschlussführung und die Deckenführung ein. Dadurch kann im Inneren des Gehäuses ein Behandlungsraum bereitgestellt werden, in welchem ein zur Behandlung der Behälterverschlüsse erforderliches oder erwünschtes Klima gezielt eingestellt werden kann. Bevorzugt wird ein Behandlungsmedium, bevorzugt ein Desinfektionsmedium, besonders bevorzugt H2O2, in das Innere des Gehäuses geleitet.

Gemäß einer weiteren bevorzugten Ausführungsform ist eine Verschlusszufuhr zum Zuführen von Behälterverschlüssen auf die Transportscheibe, bevorzugt eine getaktete Verschlusszufuhr zum getakteten Zuführen der Behälterverschlüsse, vorgesehen. Dadurch ist es möglich, Behälterverschlüsse der Transportscheibe gezielt zuzuführen.

In einer weiteren bevozugten Ausführungsform ist ein Auslass zum Abführen von Behälterverschlüssen von der Transportscheibe, bevorzugt ein getakteter Auslass zum getakteten Ausführen der Behälterverschlüsse, vorgesehen. Dadurch ist es möglich, Behälterverschlüsse von der Transportscheibe gezielt abzuführen.

Entsprechend einer weiteren bevorzugten Ausführungsform ist eine Mehrzahl von Auslässen zum Abführen von Behälterverschlüssen von der Transportscheibe vorgesehen, wobei bevorzugt jeder der Auslässe für einen bestimmten Behälterverschlusstyp vorgesehen ist. Damit kann die Vorrichtung zum Behandeln unterschiedlicher Behälterverschlusstypen verwendet werden, wobei dadurch, dass mehrere Auslässe vorgesehen sind, eine die Vorrichtung aufweisenden Anlage derart ausgebildet werden kann, dass ein Umrüsten lediglich in der Vorrichtung notwendig ist. Insbesondere bei Reinraumanlagen oder Aspetikanlagen kann dabei auf ein Verletzen der Reinraumatmosphäre in einer Verschließerkammer, in welcher befüllte Behälter mit den Behälterverschlüssen verschlossen werden, verzichtet werden.

Damit kann jedem Auslass entsprechend eine dem jeweiligen Behälterverschlusstyp zugeordnetes nachgelagerte Transportvorrichtung vorgesehen sein - beispielsweise eine Transportschiene - mittels welcher die Behälterverschlüsse dann der nachfolgenden Behandlung - beispielsweise dem Verschließer - zugefördert werden.

Gemäß einer weiteren bevorzugten Ausführungsform ist eine Ausleiteinrichtung zum Zuführen der Behälterverschlüsse von der Verschlussführung zu dem mindestens einen Auslass zwischen der Verschlussführung und dem mindestens einen Auslass vorgesehen.

Um eine unkomplizierte Umrüstung der Vorrichtung von der Behandlung eines Verschlusstyps auf einen anderen Verschlusstyp zu ermöglichen, kann die Ausleiteinrichtung gemäß einer weiteren bevorzugten Ausführungsform ein auswechselbares Einsatzteil aufweisen, wobei bevorzugt Einsetzteile unterschiedlicher Form in die Ausleiteinrichtung einsetzbar sind, wobei jedes Einsetzteil zum Zuführen der Behälterverschlüsse an einen bestimmten Auslass ausgebildet sind. Bevorzugt ist jeder der Auslässe für einen oder mehrere bestimmte Behälterverschlusstypen vorgesehen. Je nachdem, welcher Behälterverschlusstyp behandelt werden soll, wird ein entsprechendes Einsatzteil eingesetzt, dass die Behälterverschlüsse jeweils den zum Auslassen des jeweiligen Behältertyps vorgesehenen Auslass zugeführt werden.

Wenn die Ausleiteinrichtung entsprechend einer weiteren bevorzugten Ausführungsform ein verstellbares Weichenelement aufweist, wobei das Weichenelement zwischen einer Mehrzahl von Weichenpositionen verstellbar ist, wobei das Weichenelement in jeder der Weichenpositionen die Behälterverschlüsse einem anderen Auslass zuführt, kann die Vorrichtung auf einfacher Weise von der Behandlung von Behälterverschlüssen eines Typs auf die Behandlung von Behälterverschlüsse eines anderen Typs umgerüstet werden. Die Umrüstung kann dabei insbesondere durch ein einfaches Verstellen der Weiche, bevorzugt infolge eines Steuerbefehls, erfolgen. Ein Eingriff in beziehungsweise ein Öffnen des Gehäuses der Vorrichtung ist mithin nicht erforderlich.

Um Verunreinigungen aus der Vorrichtung ausleiten zu können, kann gemäß einer weiteren bevorzugten Ausführungsform an einem Bodenbereich des Gehäuses ein Ablauf angeordnet sein, wobei der Ablauf bevorzugt im Bereich eines Auslasses angeordnet ist und der Ablauf bevorzugt derart ausgebildet ist, dass abzulassende Partikel und/oder Flüssigkeiten in einen an die Vorrichtung anschließenden Isolator ablassbar sind. Bevorzugt ist der Ablauf zusammen mit einem Auslass zum Übergeben der Behälterverschlüsse von der Vorrichtung an eine der Vorrichtung nachgelagerten Behandlungsvorrichtung, bevorzugt an einen Verschließer zum Verschließen von Behältern mit den Behälterverschlüssen beziehungsweise eine Transportvorrichtung zum Transportieren der Behälterverschlüsse zu der Verschließvorrichtung, ausgebildet. Mithin ist dadurch kein separater Auslass vorzusehen, die abzuführenden Stoffe können ferner in die die weitere Behandlungsvorrichtung einhausende Kammer abgeführt werden.

Gemäß einer weiteren bevorzugten Ausführungsform sind eine Schwallöffnung zum Einlassen eines Spülmediums und/oder ein Gaseinlass zum Einlassen eines Behandlungsmediums, bevorzugt eines Behandlungsgases, besonders bevorzugt H2O2, und/oder ein Gauauslass zum Abführen des Behandlungsmediums , bevorzugt Behandlungsgases, besonders bevorzugt H2O2, vorgesehen. Dadurch kann ein Spülen der Vorrichtung beziehungsweise ein Strom von Behandlungsmedium erzielt werden.

Um ein besonders sicheres Führen der Behälterverschlüsse durch die Verschlussführung und/oder die Deckenführung zu erzielen, können die Verschlussführung und/oder die Deckenführung entsprechend einer weiteren bevorzugten Ausführungsform ein durchgängiges, bevorzugt einstückiges, Bandmaterial, bevorzugt ein Blechband, aufweisen.

Der Durchsatz beziehungsweise die Leistung der Vorrichtung kann auf einfache Weise erhöht werden, wenn gemäß einer weiteren bevorzugten Ausführungsform eine Mehrzahl von Verschlussführungen vorgesehen ist, wobei bevorzugt jede Verschlussführung eine separat höhenverstellbare Deckenführung aufweist.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine perspektivische Seitenansicht einer Vorrichtung zum Behandeln von Behälterverschlüssen gemäß einer ersten Ausführungsform;
- Figur 2: schematisch eine perspektivische Schnitt-Detailansicht der Vorrichtung aus Figur 1;
- Figur 3: schematisch eine Schnittansicht der Vorrichtung aus Figur 1;
- Figur 4: schematisch eine weitere Schnittansicht der Vorrichtung aus Figur 1;
- Figur 5: schematisch eine weitere Schnittansicht der Vorrichtung aus Figur 1; und
- Figur 6: schematisch eine Schnittansicht einer Vorrichtung zum Behandeln von Behälterverschlüssen gemäß einer weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

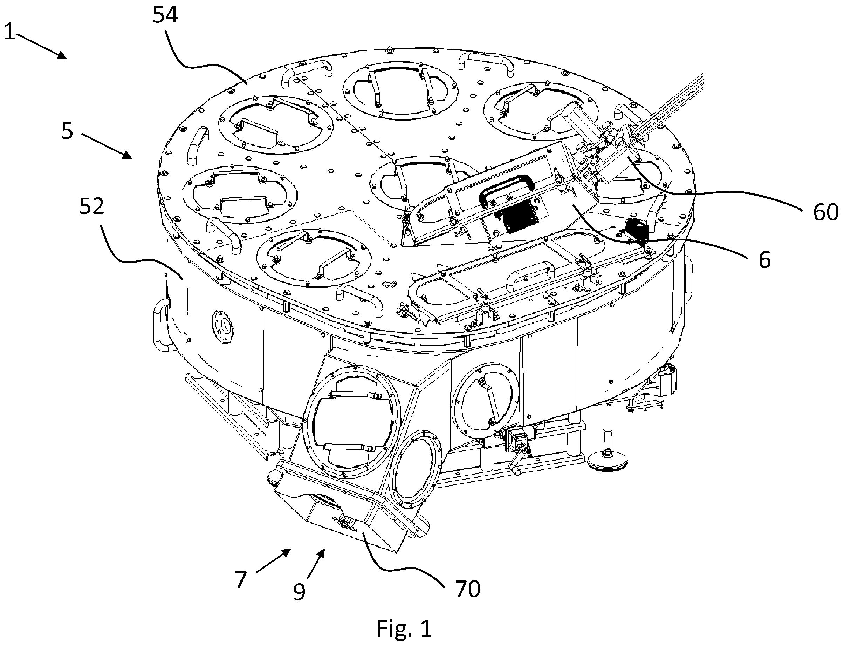

In Figur 1 ist schematisch eine perspektivische Seitenansicht einer Vorrichtung 1 zum Behandeln von Behälterverschlüssen gemäß einer ersten Ausführungsform gezeigt. Die Vorrichtung 1 weist ein abgedichtetes Gehäuse 5 auf, welches einen Boden (nicht gezeigt), eine Seitenwand 52 und einen abnehmbaren Deckel 54 umfasst. Zum Zuführen von zu behandelnden Behälterverschlüssen in die Vorrichtung 1 ist an dem Deckel 54 eine getaktete Verschlusszufuhr 6 angeordnet. Die Verschlusszufuhr 6 erhält über eine Verschlussrutsche 62 von einer der Vorrichtung 1 vorgelagerten Verschlusssortiervorrichtung (nicht gezeigt) sortierte, zu behandelnde Behälterverschlüsse. Die Verschlusszufuhr 6 weist eine Schleuse 60 auf, mittels welcher die zu behandelnden Behälterverschlüsse einzeln eingeschleust werden.

In dem Gehäuse 5 ist ferner eine Auslassöffnung 70 angeordnet, in welcher ein Auslass 7 zum Abführen behandelter Behälterverschlüsse aus der Vorrichtung 1 angeordnet ist. In einer Getränkeabfüllanlage kann die Auslassöffnung 70 beziehungsweise der Auslass 7 mit einer weiteren Transporteinrichtung (nicht gezeigt) dichtend verbunden werden, welche die behandelten Behälterverschlüsse einem Behälterverschließer zuführt.

Die Auslassöffnung 70 des Auslasses 7 erstreckt sich teilweise über einen Bodenbereich des Gehäuses 5, wobei die Auslassöffnung 70 ferner als Ablauf 9 zum Ablassen von abzulassenden Partikeln und/oder Flüssigkeiten aus dem Gehäuse 5 vorgesehen ist. Die abzulassenden Partikel und/oder Flüssigkeiten sind dadurch in einen an die Vorrichtung 1 anschließenden Isolator ablassbar.

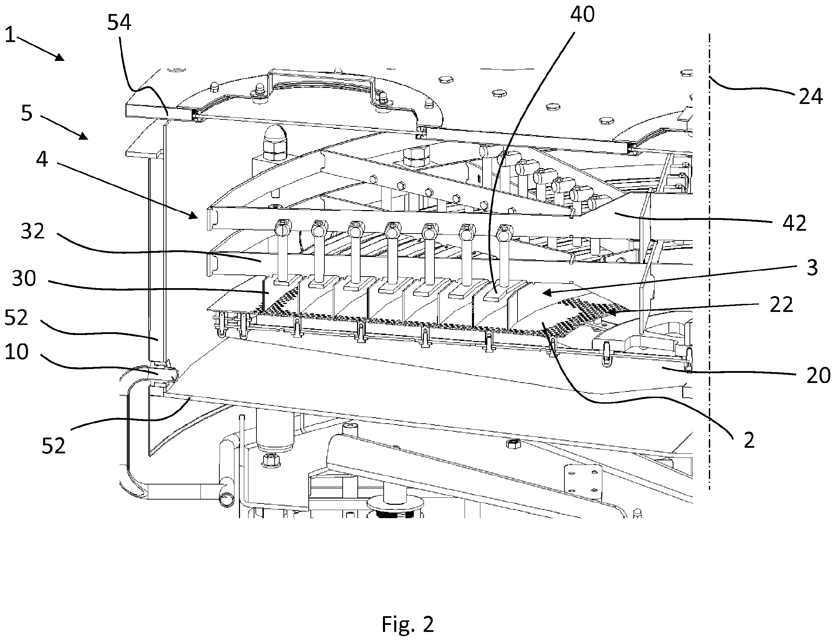

Figur 2 zeigt schematisch eine perspektivische Schnitt-Detailansicht der Vorrichtung 1 aus Figur 1. Das Gehäuse 5 haust eine um eine vertikale Drehachse 24 drehbare Transportscheibe 2 zum Transportieren der Behälterverschlüsse und eine oberhalb der Transportscheibe 2 angeordnete, spiralförmige Verschlussführung 3 zum seitlichen Führen der Behälterverschlüsse ein. Die Verschlussführung 3 weist ein durchgängiges, bevorzugt einstückig ausgebildetes, spiralförmiges Blechband 30 auf, welches an einer Mehrzahl von Trägern 32 fest angeordnet ist. Unterhalb des spiralförmigen Blechbands 30 ist die Transportscheibe 2 angeordnet, welche eine Vielzahl von gleichmäßig verteilten Perforationen 22 aufweist.

Ferner ist eine höhenverstellbare Deckenführung 4 zum Führen der Behälterverschlüsse von oben vorgesehen. Die Deckenführung 4 weist ein spiralförmig ausgebildetes, flaches Führungsblech 40 auf, welches an einer in Richtung der Drehachse 24 höhenverstellbaren Halterung 42 befestigt ist. Durch ein Verschieben der Halterung 42 in Richtung der Drehachse 24 wird ein zwischen dem Führungsblech 40 und der Transportscheibe 2 vorliegender Abstand verändert.

Zum Behandeln der zu behandelnden Behälterverschlüsse wird die Transportscheibe 2 in Rotation versetzt. Zu behandelnde Behälterverschlüsse werden über die Verschlusszufuhr 6 (siehe Figur 1) in einen Anfangsbereich der Verschlussführung 4 nahe der Drehachse 24 an die Transportscheibe 2 zugeführt. Durch die Drehbewegung der Transportscheibe 2 erfahren die sich auf der Transportscheibe 2 befindlichen Behälterverschlüsse ein Abrollen an der Verschlussführung 3 entlang einer durch die Form der Verschlussführung 3 vorgegebenen Führungsbahn. Mithin erfahren die Behälterverschlüsse in Bezug auf die Drehachse 24 einen Transport von radial innen nach radial außen. Durch den Transport der Behälterverschlüsse von radial innen nach außen wird der Abstand von Behälterverschluss zu Behälterverschluss mit der Verlagerung der Bahn nach außen immer größer, da mit steigenden Durchmesser auch der Umfang steigt. Mithin wird der Abstand zwischen den einzelnen Behälterverschlüssen immer größer, was einem Aufstauen von Behälterverschlüssen entgegenwirkt.

Die Deckenführung 4 ist derart angeordnet, dass das Führungsblech 40 sich zwischen dem Blechband 30 erstreckt. Durch die Deckenführung 4 ist ermöglicht, dass die Behälterverschlüsse stets in einer Position mit korrekter Orientierung, in welcher ein optimales Behandlungsergebnis erzielt werden kann, gehalten werden können. Insbesondere kann ein Kippen und/oder Verkeilen von Behälterverschlüssen vermieden werden. Die Deckenführung 4 führt die Behälterverschlüsse dabei auf der der Transportscheibe gegenübergelegenen Seite der Behälterverschlüsse. Erfährt ein entlang der Verschlussführung 3 transportierter Behälterverschluss ein Kippmoment, welches ihn aus seiner korrekt orientierten Position heraus kippen würde, so ist durch die Deckenführung 4 eine aus dem Kippmoment resultierende Kippbewegung des Behälterverschlusses vermieden. Die Behälterverschlüsse werden mithin sowohl seitlich durch die Verschlussführung 3 als auch von oben mittels der Deckenführung 4 geführt.

Durch die Perforationen 22 der Transportscheibe 2 fallen Verunreinigungen von der Transportscheibe 2 auf den darunter angeordneten Boden 52 des Gehäuses 5. Nahe des Bodens 52 ist in der Seitenwand 54 eine Schwallöffnung 10 zum Einlassen eines Spülmediums angeordnet. Durch das eingelassene Spülmedium wird der Boden 52 gespült und die Verunreinigungen, beispielsweise Partikel und/oder Flüssigkeiten, werden in Richtung des und durch den Ablauf 9 (siehe Figur 1) ausgespült. Um ein vorteilhaftes Ablaufen zu gewährlisten, ist der Boden 52 leicht, vorliegend um etwa 3°, in Richtung des Ablaufs 9 geneigt. Der Boden 52 weist mithin keine vertikalen Flächen auf, auf denen Wasser oder Reinigungsmittel liegen bleiben kann. Hierdurch kann ferner die Trocknungszeit der Vorrichtung 1 nach einer Reinigung kurz gehalten werden.

In dem Gehäuse 5 sind weiterhin eine Vielzahl von nicht gezeigten Gaseinlässen zum Einlassen eines Behandlungsgases, vorliegend H2O2, und eine Vielzahl von nicht gezeigten Gasauslässen zum Abführen des Behandlungsgases, vorliegend H2O2, vorgesehen. Durch das Vorsehen der Gaseinlässe und Gasauslässe kann ein Behandlungsgasstrom durch das Gehäuseinnere erzeugt werden, wobei die auf der Transportscheibe 2 befindlichen Behälterverschlüsse mit dem Behandlungsgasstrom in Kontakt kommen und dadurch desinfiziert werden. Der prinzipielle Aufbau der Gaseinlässe und Gasauslässe ist an sich bekannt und kann beispielsweise der EP 2 039 439 A1 entnommen werden.

In einer alternativen Ausführungsform ist nur ein Gaseinlass, bevorzugt im Bereich der Mitte des Gehäuses 5, vorgesehen. Der Gaseinlass kann beispielsweise in der Nähe der Verschlusszufuhr 6 vorgesehen.

Einer oder mehrere Gasauslässe können im Bereich der Auslässe 7, 7', 7" vorgesehen sein.

Die Drehgeschwindigkeit der Transportscheibe 2 ist einstellbar, so dass eine Verweilzeit der zu behandelnden Behälterverschlüsse 11 einstellbar ist.

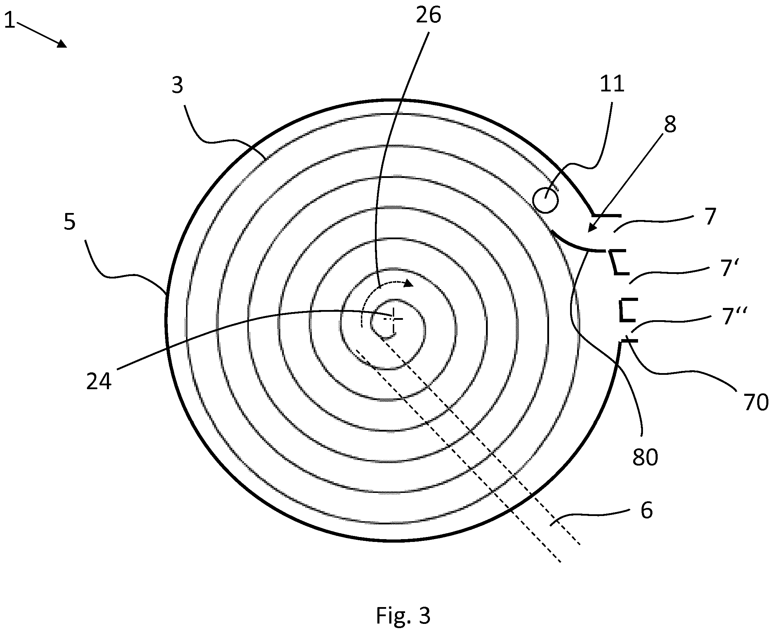

Aus Figur 3 ist schematisch eine Schnittansicht der Vorrichtung 1 aus Figur 1 zu entnehmen. Wie bereits oben beschrieben, führt die Verschlusszufuhr 6 die zu behandelnden Behälterverschlüsse 11 in einen Mittelbereich der Verschlussführung 3. Durch die Drehbewegung, welche hier mit dem Bezugszeichen 26 angedeutet ist, der Transportscheibe 2 (nicht gezeigt) um die Drehachse 24 werden die Behälterverschlüsse 11 entlang der spiralförmigen Verschlussführung 3 in Bezug auf die Drehachse 24 von radial innen nach radial außen bewegt, wobei sie während des Transports mit dem Behandlungsgas in Kontakt kommen. Sie gelangen dann zu einer Ausleiteinrichtung 8, die zwischen der Verschlussführung 3 und einer Mehrzahl von Auslässen 7, 7', 7" zum Ausführen der Behälterverschlüsse 11 von der Transportscheibe 2 und aus dem Gehäuse 5 heraus angeordnet ist. Jeder der Auslässe 7, 7', 7" ist dabei zum Ausleiten von Behälterverschlüssen eines bestimmten Behälterverschlusstyps vorgesehen.

In einer bevorzugten Ausgestaltung kann die Ausleiteinrichtung 8 die Behälterverschlüsse auch getaktet ausgeben.

Die Ausleiteinrichtung 8 weist vorliegend ein erstes Einsatzteil 80 auf, welches zum Zuführen von Behälterverschlüssen 11 eines ersten Typs an einen ersten Auslass 7 ausgebildet ist. Das Einsatzteil 8 ist auswechselbar ausgebildet. Es kann, wenn die Vorrichtung 1 zum Behandeln von Behälterverschlüssen 11 eines anderen Typs umgerüstet werden soll, entfernt und durch ein anderes Einsatzteil ersetzt werden.

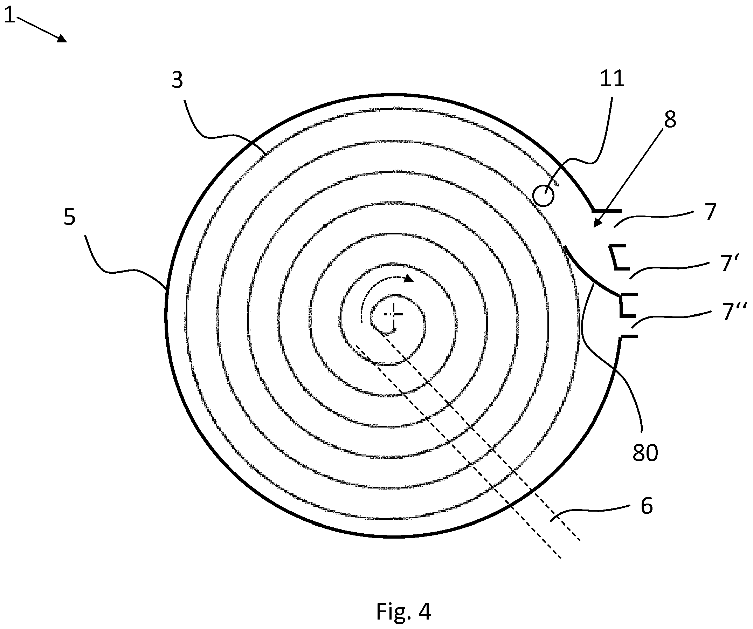

In Figur 4 ist schematisch die Vorrichtung 1 aus Figur 1 nach dem Umrüsten zum Behandeln von Behälterverschlüssen 11 eines zweiten Typs in einer Schnittansicht gezeigt. Anstelle des ersten Einsatzteils 80 weist die Ausleiteinrichtung 8 nun ein zweites Einsatzteil 80' auf, welches hinsichtlich seiner Position in der Vorrichtung 1 und seiner Form von dem ersten Einsatzteil 80 unterschiedlich ist. Das zweite Einsatzteil 80' ist mithin zum Zuführen von Behälterverschlüssen 11 eines zweiten Typs an einen zweiten Auslass 7' ausgebildet.

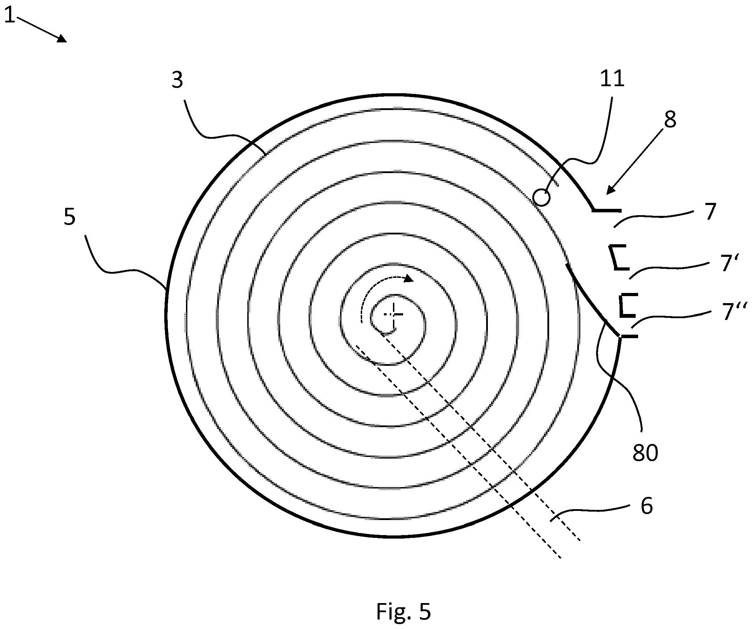

In Figur 5 ist schematisch die Vorrichtung 1 aus Figur 1 nach einem erneuten Umrüsten zum Behandeln von Behälterverschlüssen 11 eines dritten Typs in einer Schnittansicht gezeigt. Anstelle des ersten oder zweiten Einsatzteils 80, 80' weist die Ausleiteinrichtung 8 nun ein drittes Einsatzteil 80" auf, welches hinsichtlich seiner Position in der Vorrichtung 1 und seiner Form von dem ersten und zweiten Einsatzteil 80, 80' unterschiedlich ist. Das dritte Einsatzteil 80" ist mithin zum Zuführen von Behälterverschlüssen 11 eines dritten Typs an einen dritten Auslass 7" ausgebildet.

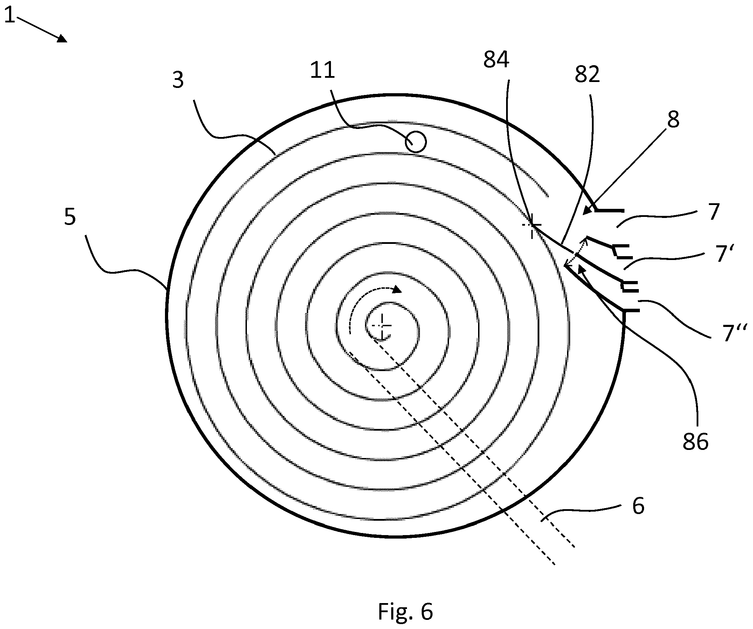

In Figur 6 ist schematisch eine Schnittansicht einer Vorrichtung 1 zum Behandeln von Behälterverschlüssen 11 gemäß einer weiteren Ausführungsform gezeigt. Die Vorrichtung 1 entspricht in ihrem Aufbau im Wesentlichen der Vorrichtung 1 aus den Figuren 1 bis 5, wobei die Vorrichtung 1 gemäß des in Figur 6 gezeigten Ausführungsbeispiels eine Ausleiteinrichtung 8 aufweist, die statt unterschiedlicher, auszutauschender Einsatzteile ein verstellbares Weichenelement 82 aufweist, wobei das Weichenelement 82 innerhalb eines Schwenkbereichs 86 zwischen einer Mehrzahl von Weichenpositionen um eine Schwenkachse 84 verschwenkbar ist, wobei das Weichenelement 82 in jeder der Weichenpositionen die Behälterverschlüsse 11 jeweils einem anderen Auslass 7, 7', 7" zuführt. In Figur 6 gezeigt ist die Weiche 82 in einer zweiten Weichenposition positioniert, so dass die Behälterverschlüsse 11 dem zweiten Auslass 7' zugeführt werden. Zum Umrüsten der Vorrichtung für das Behandeln von Behälterverschlüssen 11 eines anderen Typs ist lediglich die Weiche 82 in eine andere Weichenposition zu verstellen. Ein Öffnen des Gehäuses 5 zum Austauschen zweier Einsatzteile ist nicht erforderlich.

### Bezugszeichenliste

- 1: Vorrichtung
- 2: Transportscheibe
- 20: Trägerarm
- 22: Perforation
- 24: Drehachse
- 26: Drehbewegung
- 3: Verschlussführung
- 30: Blechband
- 32: Träger
- 4: Deckenführung
- 40: Führungsblech
- 42: Halterung
- 5: Gehäuse
- 50: Boden
- 52: Seitenwand
- 54: Deckel
- 6: Verschlusszufuhr
- 60: Schleuse
- 62: Verschlussrutsche
- 7: Auslass
- 70: Auslassöffnung
- 8: Ausleiteinrichtung
- 80: Einsatzteil
- 82: Weiche
- 84: Schwenkachse
- 86: Schwenkbereich
- 9: Ablauf
- 10: Schwallöffnung
- 11: Behälter

## Patentansprüche

1. Vorrichtung (1) zum Behandeln von Behälterverschlüssen (11), bevorzugt zum Desinfizieren von Behälterverschlüssen (11) in einer Getränkeabfüllanlage, umfassend eine um eine vertikale Drehachse (24) drehbare Transportscheibe (2) zum Transportieren der Behälterverschlüsse (11) und eine oberhalb der Transportscheibe (2) angeordnete, spiralförmige Verschlussführung (3) zum seitlichen Führen der Behälterverschlüsse (11), wobei
die Verschlussführung (3) eine Deckenführung (4) zum Führen der Behälterverschlüsse (11) von oben aufweist,
**dadurch gekennzeichnet, dass**
die Deckenführung (4) ein spiralförmig ausgebildetes, flaches Führungsblech (40) aufweist, welches an einer in Richtung der Drehachse (24) höhenverstellbaren Halterung (42) befestigt ist.

2. Vorrichtung (1) gemäß Anspruch 1, **dadurch gekennzeichnet, dass** die Deckenführung (4) stufenlos höhenverstellbar ist.

3. Vorrichtung (1) gemäß Anspruch 1 oder 2, **dadurch gekennzeichnet, dass** die Verschlussführung (3) feststehend ist und/oder **dass** die Transportscheibe (2) perforiert ist, wobei die Transportscheibe (2) bevorzugt eine Vielzahl von bevorzugt gleichmäßig verteilten Perforationen (22) aufweist.

4. Vorrichtung (1) gemäß einem der vorstehenden Ansprüche, **dadurch gekennzeichnet, dass** die Verschlussführung (3) derart ausgebildet ist, dass in Bezug auf die Drehachse (24) die Behälterverschlüsse (11) von radial innen nach radial außen transportierbar sind.

5. Vorrichtung (1) gemäß einem der vorstehenden Ansprüche, **dadurch gekennzeichnet, dass** eine Drehgeschwindigkeit der Transportscheibe (2) einstellbar ist, wobei bevorzugt durch ein Einstellen der Drehgeschwindigkeit der Transportscheibe (2) eine Verweilzeit eines zu behandelnden Behälterverschlusses (11) einstellbar ist.

6. Vorrichtung gemäß einem der vorstehenden Ansprüche, **dadurch gekennzeichnet, dass** ein Gehäuse (5), bevorzugt ein abgedichtetes Gehäuse (5), die Transportscheibe (2), die Verschlussführung (3) und die Deckenführung (4) einhaust.

7. Vorrichtung (1) gemäß einem der vorstehenden Ansprüche, **dadurch gekennzeichnet, dass** eine Verschlusszufuhr (6) zum Zuführen von Behälterverschlüssen (11) auf die Transportscheibe (2), bevorzugt eine getaktete Verschlusszufuhr (6) zum getakteten Zuführen der Behälterverschlüsse (11), vorgesehen ist und/oder ein Auslass (7) zum Abführen von Behälterverschlüssen (11) von der Transportscheibe (2), bevorzugt ein getakteter Auslass (7) zum getakteten Ausführen der Behälterverschlüsse (11), vorgesehen ist.

8. Vorrichtung (1) gemäß einem der vorstehenden Ansprüche, **dadurch gekennzeichnet, dass** eine Mehrzahl von Auslässen (7,7',7") zum Abführen von Behälterverschlüssen (11) von der Transportscheibe (2) vorgesehen ist, wobei bevorzugt jeder der Auslässe (7,7',7") für einen bestimmten Behälterverschlusstyp vorgesehen ist.

9. Vorrichtung (1) gemäß Anspruch 7 oder 8, **dadurch gekennzeichnet, dass** eine Ausleiteinrichtung (8) zum Zuführen der Behälterverschlüsse (11) von der Verschlussführung (3) zu dem mindestens einen Auslass (7) zwischen der Verschlussführung (3) und dem mindestens einen Auslass (7) vorgesehen ist.

10. Vorrichtung (1) gemäß Anspruch 9, **dadurch gekennzeichnet, dass** die Ausleiteinrichtung (8) ein auswechselbares Einsatzteil (80) aufweist, wobei bevorzugt Einsetzteile (80,80',80") unterschiedlicher Form in die Ausleiteinrichtung (8) einsetzbar sind, wobei jedes Einsetzteil (80,80',80") zum Zuführen der Behälterverschlüsse (11) an einen bestimmten Auslass (7,7',7") ausgebildet sind.

11. Vorrichtung (1) gemäß Anspruch 9 oder 10, **dadurch gekennzeichnet, dass** die Ausleiteinrichtung (8) ein verstellbares Weichenelement (82) aufweist, wobei das Weichenelement (82) zwischen einer Mehrzahl von Weichen positionen verstellbar ist, wobei das Weichenelement (82) in jeder der Weichenpositionen die Behälterverschlüsse (11) jeweils einem anderen Auslass (7,7',7") zuführt.

12. Vorrichtung (1) gemäß einem der Ansprüche 6 bis 11, **dadurch gekennzeichnet, dass** an einem Bodenbereich des Gehäuses (5) ein Ablauf (9) angeordnet ist, wobei der Ablauf (9) bevorzugt im Bereich eines Auslasses (7) angeordnet ist, wobei der Ablauf (9) bevorzugt derart ausgebildet ist, dass abzulassende Partikel und/oder Flüssigkeiten in einen an die Vorrichtung (1) anschließenden Isolator ablassbar sind.

13. Vorrichtung (1) gemäß einem der vorstehenden Ansprüche, **dadurch gekennzeichnet, dass** eine Schwallöffnung (10) zum Einlassen eines Spülmediums und/oder ein Gaseinlass zum Einlassen eines Behandlungsgases, bevorzugt H2O2, und/oder ein Gauauslass zum Abführen des Behandlungsgases, bevorzugt H2O2, vorgesehen ist.

14. Vorrichtung (1) gemäß einem der vorstehenden Ansprüche, **dadurch gekennzeichnet, dass** die Verschlussführung (3) und/oder die Deckenführung (4) ein durchgängiges, bevorzugt einstückiges, Bandmaterial, bevorzugt ein Blechband, aufweist.

15. Vorrichtung (1) gemäß einem der vorstehenden Ansprüche, **dadurch gekennzeichnet, dass** eine Mehrzahl von Verschlussführungen (3) vorgesehen ist, wobei bevorzugt jede Verschlussführung (3) eine separat höhenverstellbare Deckenführung (4) aufweist.

## Claims

1. Device (1) for the treatment of container closures (11), preferably for disinfecting container closures (11) in a beverage filling plant, comprising a transport plate (2) rotatable about a vertical rotational axis (24) for transporting the container closures (11) and a spiral-shaped closure guide (3), arranged above the transport plate (2), for the lateral guidance of the container closures (11), wherein
the closure guide (3) has a ceiling guide (4) for guiding the container closures (11) from above,
**characterised in that**
the ceiling guide (4) has a spiral-shaped flat guide sheet (40) which is fastened to a holder (42) which is height-adjustable in the direction of the rotational axis (24).

2. Device (1) according to claim 1, **characterised in that** the ceiling guide (4) is steplessly height-adjustable.

3. Device (1) according to claim 1 or 2, **characterised in that** the closure guide (3) is fixed and/or that the transport plate (2) is perforated, wherein the transport plate (2) preferably has a multiplicity of preferably evenly distributed perforations (22).

4. Device (1) according to any of the preceding claims, **characterised in that** the closure guide (3) is configured such that in relation to the rotational axis (24) container closures (11) are transportable from radially inside to radially outside.

5. Device (1) according to any of the preceding claims, **characterised in that** a rotational speed of the transport plate (2) can be set, wherein preferably by means of setting the rotational speed of the transport plate (2) a dwell time of a container closure (11) to be treated is settable.

6. Device according to any of the preceding claims, **characterised in that** a housing (5), preferably a sealed housing (5), contains the transport plate (2), the closure guide (3) and the ceiling guide (4).

7. Device (1) according to any of the preceding claims, **characterised in that** a closure feed (6) for supplying container closures (11) is provided on the transport plate (2), preferably a clocked closure feed (6) for the clocked supply of the container closures (11) and/or an outlet (7) for discharging container closures (11) from the transport plate (2), preferably a clocked outlet (7) for the clocked discharged of container closures (11) is provided.

8. Device (1) according to any of the preceding claims, **characterised in that** a plurality of outlets (7, 7', 7") is provided for discharging container closures (11) from the transport plate (2), wherein preferably each of the outlets (7, 7', 7") is provided for a specific type of container closure.

9. Device (1) according to claim 7 or 8, **characterised in that** a discharging means (8) is provided for feeding the container closures (11) from the closure guide (3) to the at least one outlet (7) between the closure guide (3) and the at least one outlet (7).

10. Device (1) according to claim 9, **characterised in that** the discharging means (8) has an exchangeable insert part (80), wherein preferably insert parts (80, 80', 80") of different shapes can be inserted into the discharging means (8), wherein each insert part (80, 80', 80") is configured to feed the container closures (11) to a specific outlet (7, 7', 7").

11. Device (1) according to claim 9 or 10, **characterised in that** the discharging means (8) has an adjustable switch element (82), wherein the switch element (82) is adjustable between a plurality of switch positions wherein the switch element (82) in each of the switch positions supplies the container closures (11) to in each case another outlet (7, 7', 7").

12. Device (1) according to any of claims 6 to 11, **characterised in that** at a floor region of the housing (5) is arranged a drain (9), wherein the drain (9) is preferably arranged in the region of an outlet (7), wherein the drain (9) is preferably designed such that particles and/or liquids to be discharged can be discharged into an isolator connected to the device (1).

13. Device (1) according to any of the preceding claims, **characterised in that** a surge opening (10) for letting in a flushing agent and/or a gas inlet for letting in a treatment gas, preferably H2O2, and/or a gas outlet for discharging the treatment gas, preferably H2O2, is provided.

14. Device (1) according to any of the preceding claims, **characterised in that** the closure guide (3) and/or the ceiling guide (4) has a continuous, preferably integral strip material, preferably a metal strip.

15. Device (1) according to any of the preceding claims, **characterised in that** a plurality of closure guides (3) is provided, wherein preferably each closure guide (3) has a separate height-adjustable ceiling guide (4).

## Revendications

1. Dispositif (1) de traitement de fermetures de récipient (11), de préférence de désinfection de fermetures de récipient (11) dans une installation de mise en bouteille de boisson, comprenant un disque de transport (2) pouvant tourner autour d'un axe de rotation vertical (24) pour transporter des fermetures de récipient (11) et un guide de fermeture hélicoïdal (3) agencé au-dessus du disque de transport (2) et permettant de guider latéralement les fermetures de récipient (11), dans lequel
le guide de fermeture (3) présente un guide supérieur (4) destiné au guidage des fermetures de récipient (11) par le haut,
**caractérisé en ce que,**
le guide supérieur (4) présente une plaque de guidage plate réalisée de forme hélicoïdale (40) qui est fixée à un support (42) réglable en hauteur dans la direction de l'axe de rotation (24).

2. Dispositif (1) selon la revendication 1, **caractérisé en ce que** la hauteur du guide supérieur (4) est réglable en continu.

3. Dispositif (1) selon la revendication 1 ou 2, **caractérisé en ce que** le guide de fermeture (3) est fixe et/ou **en ce que** le disque de transport (2) est perforé, le disque de transport (2) présentant de préférence une pluralité de perforations (22) de préférence réparties uniformément.

4. Dispositif (1) selon l'une des revendications précédentes, **caractérisé en ce que** le guide de fermeture (3) est conçu de telle manière que les fermetures de récipient (11) peuvent être transportées de radialement vers l'intérieur à radialement vers l'extérieur par rapport à l'axe de rotation (24).

5. Dispositif (1) selon l'une des revendications précédentes, **caractérisé en ce qu'** une vitesse de rotation du disque de transport (2) peut être réglée, un temps de séjour d'une fermeture de récipient (11) à traiter pouvant de préférence être réglé en réglant la vitesse de rotation du disque de transport (2).

6. Dispositif selon l'une des revendications précédentes, **caractérisé en ce qu'**un boîtier (5), de préférence un boîtier étanche (5), renferme le disque de transport (2), le guide de fermeture (3) et le guide supérieur (4).

7. Dispositif (1) selon l'une des revendications précédentes, **caractérisé en ce qu'** une alimentation de fermetures (6) pour alimenter les fermetures de récipient (11) sur le disque de transport (2) est prévue, de préférence une alimentation de fermetures cadencée (6) pour une alimentation cadencée des fermetures de récipient (11), et/ou une sortie (7) pour évacuer les fermetures de récipient (11) à partir du disque de transport (2) est prévue, de préférence une sortie cadencée (7) pour une évacuation cadencée des fermetures de récipient (11).

8. Dispositif (1) selon l'une des revendications précédentes, **caractérisé en ce que** plusieurs sorties (7, 7', 7") sont prévues pour retirer les fermetures de récipient (11) à partir du disque de transport (2), dans lequel de préférence chacune des sorties (7, 7', 7") est prévue pour un type de fermeture de récipient spécifique.

9. Dispositif (1) selon la revendication 7 ou 8, **caractérisé en ce qu'** un dispositif de redirection (8) pour alimenter les fermetures de récipient (11) du guide de fermeture (3) jusqu'à la au moins une sortie (7) est prévu entre le guide de fermeture (3) et la au moins une sortie (7).

10. Dispositif (1) selon la revendication 9, **caractérisé en ce que** le dispositif de redirection (8) présente une partie d'insert interchangeable (80), de préférence des parties d'insertion (80, 80', 80") de différentes formes pouvant être insérées dans le dispositif de redirection (8), chaque partie d'insertion (80, 80', 80") étant conçue pour alimenter les fermetures de récipient (11) vers une sortie spécifique (7, 7', 7").

11. Dispositif (1) selon la revendication 9 ou 10, **caractérisé en ce que** le dispositif de redirection (8) présente un élément d'aiguillage réglable (82), l'élément d'aiguillage (82) étant réglable entre plusieurs positions d'aiguillage, l'élément d'aiguillage (82), dans chacune des positions de commutation, alimente les fermetures de récipient (11) chacune vers une sortie différente (7, 7', 7").

12. Dispositif (1) selon l'une des revendications 6 à 11, **caractérisé en ce qu'** un déversoir (9) est agencé au niveau d'une zone inférieure du boîtier (5), le déversoir (9) étant de préférence agencé dans la zone d'une sortie (7), le déversoir (9) étant de préférence conçu de telle manière que les particules et/ou les liquides à évacuer peuvent être évacués dans un isolateur relié au dispositif (1).

13. Dispositif (1) selon l'une des revendications précédentes, **caractérisé en ce qu'**une ouverture anti-retour (10) pour l'admission d'un fluide de rinçage et/ou une entrée de gaz pour l'admission d'un gaz de traitement, de préférence H2O2, et/ou une sortie de manomètre pour l'évacuation du gaz de traitement, de préférence H2O2, est/sont prévue(s).

14. Dispositif (1) selon l'une des revendications précédentes, **caractérisé en ce que** le guide de fermeture (3) et/ou le guide supérieur (4) présente(nt) un matériau en bande continue, de préférence monobloc, de préférence une bande de tôle.

15. Dispositif (1) selon l'une des revendications précédentes, **caractérisé en ce qu'**une pluralité de guides de fermeture (3) est prévue, chaque guide de fermeture (3) présentant de préférence un guide supérieur (4) réglable en hauteur séparé.
